# EUROPÄISCHE PATENTSCHRIFT

(11) **EP 2 118 495 B1**
(45) Veröffentlichungstag und Bekanntmachung des Hinweises auf die Patenterteilung: **26.05.2010**
(21) Anmeldenummer: 08700012.1
(22) Anmeldetag: 02.01.2008
(51) Int. Cl.: F04D 25/08, F04D 29/58, H02K 9/20

(54) **GEBLÄSE FÜR EIN BEATMUNGSGERÄT UND BEATMUNGSGERÄT MIT EINEM SOLCHEN**
BLOWER FOR A MEDICAL VENTILATOR AND MEDICAL VENTILATOR WITH SUCH A BLOWER
SOUFFLANTE POUR UN RESPIRATEUR ET RESPIRATEUR POURVU D'UNE TELLE SOUFFLANTE

(30) Priorität: 15.02.2007 CH 2522007
(43) Veröffentlichungstag der Anmeldung: 18.11.2009
(73) Patentinhaber: Hamilton Medical AG, 7402 Bonaduz (CH)
(72) Erfinder: STEINBACHER, Peter, CH-7315 Vättis (CH)
(74) Vertreter: Hasler, Erich
(86) Internationale Anmeldenummer: PCT/CH2008/000003
(87) Internationale Veröffentlichungsnummer: WO 2008/098382

(56) Entgegenhaltungen:
- EP-A- 1 596 071
- DE-C1- 10 063 941
- DE-C1- 19 808 602
- JP-A- 57 062 754
- US-A- 2 683 823

## Beschreibung

Die Erfindung betrifft ein Gebläse für ein Beatmungs- oder Anästhesiegerät, wie auch ein Beatmungs- oder Anästhesiegerät mit einem solchen Gebläse.

Es sind Gebläse im Handel erhältlich, die für Beatmungsgeräte dahingehend geeignet sind, als sie die für Beatmungsgeräte notwendigen Volumina und Drücke liefern können. Bei der Verwendung eines solchen Gebläses in einem Beatmungsgerät sind jedoch auch Schallemission und Wärmeentwicklung beim Komprimieren des Beatmungsgases zu berücksichtigen.

Die Druckschrift DE 19888602 A wird als nächstliegender Stand der Technik angesehen.

Schallemissionen können durch Schall dämmende und Schall absorbierende Schichten reduziert werden. Schall dämmende und absorbierende Schichten behindern jedoch die Abgabe von Wärme, so dass im Innern eines leisen Gebläses eine umso grössere Hitzeentwicklung festzustellen ist.

Es ist daher die Aufgabe der Erfindung, ein Gebläse für ein Beatmungs- oder Anästhesiegeräts zu schaffen, bei dem das gelieferte Beatmungsgas ungekühlt für die Beatmung eines Patienten verwendet werden kann und die Betriebstemperatur des Elektromotors derart gering ist, dass eine lange Lebensdauer des Elektromotors gewährleistet ist. Die Konstruktion des Gebläses soll erlauben, die Schallemissionswerte des Gebläses gering zu halten, ohne dass die Vorteile bezüglich Hitzeentwicklung verloren gehen.

Diese Aufgabe wird erfindungsgemäss durch das Gebläse gemäss Anspruch 1, bzw. ein Beatmungsgerät oder Anästhesiegerät mit einem solchen Gebläse gelöst.

Das erfindungsgemässe Gebläse ist in bekannter Art mit einem Elektromotor und mit wenigstens einem vom Elektromotor angetriebenen Kompressorrad in einem Gebläsegehäuse ausgerüstet. Diese Komponenten dienen der Kompression des Beatmungsgases. Der Elektromotor liegt an einer ersten Gehäusewandung des Gebläsegehäuses an, die aus einem gut wärmeleitenden Material, insbesondere Metall, vorzugsweise Aluminium, gefertigt ist. Diese erste Gehäusewand nimmt daher Wärme vom Motor auf. An die erste Gehäusewandung unmittelbar angrenzend ist zwischen dieser ersten Gehäusewandung und einer zweiten Gehäusewandung wenigstens ein Luftführungskanal für das komprimierte Beatmungsgas vorhanden.

Bei einer nicht erfindungsgemässen Ausrüstung des Gebläses würde das durch die Komprimierung bereits erwärmte Beatmungsgas ab einer gewissen Betriebsdauer zusätzliche Wärme von der ersten Gehäusewandung aufnehmen und daher gekühlt werden müssen.

Die Merkmale des kennzeichnenden Teils des Anspruchs 1 bewirken, dass dieser Effekt jedoch nicht eintritt. Die erste Gehäusewandung des Gebläsegehäuses ist an wenigstens eine Heatpipe angeschlossen, welche mit einem Kühlelement verbunden ist. Dadurch wird Wärme an zentraler Stelle abgeführt, so dass auch nach längerem Betreiben des Gebläses sogar Wärme von der komprimierten Luft an die gekühlte erste Gehäusewand abgegeben wird. Ein Wärmetransfer vom Motor auf das Beatmungsgas ist damit unterbrochen, und dem Beatmungsgas wird Wärme entzogen.

Diese Massnahme ermöglicht das weitgehend schalldichte abschotten des Gebläses von seiner Umgebung. Um dies zu erreichen ist es erforderlich, dass die Heatpipe eine Schalldämmschicht durchdringt, die das Gebläsegehäuse umschliesst, und dass das Kühlelement ausserhalb dieser Schalldämmschicht angeordnet ist. Vorteilhaft umschliesst die Schalldämmschicht die Baueinheit aus Elektromotor und Gebläsegehäuse allseitig.

Die Schalldämmschicht ist im Innern einer Aussenhülle angeordnet. Sie ist dadurch geschützt vor Verletzungen von Aussen. Die unterschiedlichen Materialien und Materialstärken von Schalldämmschicht und Aussenhülle gewährleisten eine bessere Schalldämmung in einem grösseren Frequenzspektrum als die Summe der Dämmwerte der einzelnen Komponenten ergäbe. Innerhalb dieser Aussenhülle sind die erste und die zweite Gehäusewandung und der Elektromotor angeordnet, und ausserhalb welcher Aussenhülle ist das Kühlelement angeordnet. Die im Innern des Gebläses anfallende Wärme wird daher mit der Heatpipe durch die Wärme und Schall dämmende Schalldämmschicht hinausgeführt. Die Aussenhülle könnte als Kühlelement genutzt werden. Bevorzugt wird jedoch ein separates Kühlelement.

Zweckmässigerweise ist auf der Unterdruckseite des Kompressorrads eine Schalldämpfer-Kammer vorhanden. Diese ist durch ein Labyrinth für das Beatmungsgas gebildet. Diese Schalldämpfer-Kammer und das Labyrinth sind mit Vorteil innerhalb des von der Aussenhülle umfassten Raumes angeordnet. Dadurch bilden Schalldämpfer-Kammer, Gebläsegehäuse, Motor und Schalldämmung eine bauliche Einheit, die sehr einfach als Gebläsemodul in ein Beatmungs- oder Anästhesiegerät eingebaut werden kann.

Ein solches Beatmungsgerät oder Anästhesiegerät ist entsprechend mit einem Gebläse ausgerüstet, das zur Kompression von Beatmungsgas mit einem Elektromotor und wenigstens einem vom Elektromotor angetriebenen Kompressorrad in einem Gebläsegehäuse ausgerüstet ist. Bei dem Gebläse liegt, wie bereits weiter oben ausgeführt, der Elektromotor an einer ersten Gehäusewandung des Gebläsegehäuses an, die aus einem gut wärmeleitenden Materialgefertigt ist. An die erste Gehäusewandung unmittelbar angrenzend ist zwischen dieser ersten Gehäusewandung und einer zweiten Gehäusewandung wenigstens ein Luftführungskanal für das komprimierte Beatmungsgas vorhanden. Die erste Gehäusewandung des Gebläsegehäuses ist an wenigstens eine Heatpipe angeschlossen, welche Heatpipe mit einem Kühlelement verbunden ist. Dieses Kühlelement kann nun an einer geeigneten Stelle des Beatmungs- oder Anästhesiegeräts angeordnet und dort gekühlt werden. Mit der Kühlung des Kühlelements wird Wärme zwischen dem Luftführungskanal und dem Elektromotor entzogen. Dadurch wärmt weder das erhitzte Beatmungsgas den Elektromotor und dessen Lager, noch heizt die Wärme, die im Elektromotor anfällt, das Beatmungsgas zusätzlich auf.

Diese Gebläseausbildung erlaubt, das Gebläse komplett einzupacken, ohne dass die Wärme darin gestaut wird. Es wird daher bevorzugt, das Beatmungsgerät mit wenigstens einem Schaumstoffkörper zu versehen, in welchem das Gebläse, insbesondere das Gebläsemodul insgesamt, eingebettet ist. Die Einbettung des Gebläsemoduls in einen Schaumstoffkörper dient der zusätzlichen Schalldämmung und Vibrationsdämmung, der sturzsicheren Lagerung des Gebläses im Beatmungsgerät und der sehr einfachen Fixierung des Gebläses innerhalb des Beatmungsgeräts. Vorteilhaft ist die Aussenhülle im Schaumstoffkörper eingebettet. Diese kann eine einfache, parallelepipedische Form haben und in ihrem Innern das Gebläsemodul, bzw. das Gebläse und die zum Gebläse gehörenden Bestandteile, insbesondere Schalldämpf-Kammer, Kompressor, Elektromotor, Schalldämmung, beherbergen.

In diesem wenigstens einen Schaumistoffkörper, beziehungsweise in einer Mehrzahl von solchen, zusammenpassenden Schaumstoffkörpern, sind mit Vorteil weitere technische Bestandteile des Beatmungsgeräts oder Anästhesiegeräts angeordnet. Diese sind vom Schaumstoffkörper in der zu den anderen Bestandteilen richtigen Position gehalten. Die Anordnung innerhalb solcher Schaumstoffkörper ermöglicht einen sehr einfachen Zusammenbau des Beatmungsgeräts, und eine stosssichere Lagerung der Einzelteile. Die technischen Bestandteile werden bei der Montage in einen solchen Schaumstoffkörper eingesetzt, und mehrere solcher bestückter Schaumstoffkörper übereinander oder nebeneinander gestapelt. Mit dem Stapeln werden die notwendigen Verbindungen unter den technischen Bestandteilen ineinander gesteckt und so das Beatmungsgerät zu einer funktionierenden Einheit zusammengestellt.

Zweckmässigerweise ist ein Lüfter vorhanden, um die technischen Bestandteile, die Wärme entwickeln, zu kühlen. Dazu ist zwischen den technischen Bestandteilen des Beatmungs- oder Anästhesiegeräts und dem Schaumstoffkörper ein Kühlluft-Kanalsystem ausgebildet. Dieses hat wenigstens einen Lufteinlass um frische Umgebungsluft anzusaugen. Es hat entsprechend wenigstens einen Luftauslass, um die erwärmte Luft auszustossen.

Vorteilhaft ist das Kühlelement in diesem Kühlluft-Kanalsystem angeordnet.

Das Beatmungsgerät besitzt ein Gerätegehäuse, das den oder die Schaumstoffkörper mit den darin eingelegten technischen Bestandteilen umhüllt. Zweckmässigerweise sind mehrere Schaumstoffkörper durch ein sie umschliessendes Gerätegehäuse zusammengehalten. Dadurch sind die zwischen den Schaumstoffkörpern angeordneten technischen Bestandteile des Beatmungsgeräts verschiebefest zwischen den Schaumstoffkörpern festgehalten. Ein solcher Aufbau des Beatmungsgeräts verbindet die Vorteile einer einfachen Montage mit einer Vibrationen und Schall dämmenden Konstruktion, sowie einer stosssicheren Verpackung der Bestandteile innerhalb des Gerätegehäuses.

### Kurzbeschreibung der Figuren:

Die Figuren zeigen ein Ausführungsbeispiel der Erfindung. Es zeigt:
- Fig. 1: ein erfindungsgemässes Gebläse in einem Längsschnitt entlang der Ebene AA,
- Fig. 2: das Gebläse gemäss Figur 1 in einem Querschnitt entlang der Ebene BB.
- Fig. 3: das Gebläse gemäss Figur 1 in einem Querschnitt entlang der Ebene CC.
- Fig. 4: einen schematischen Querschnitt durch ein Beatmungsgerät mit einem solchen Gebläse.

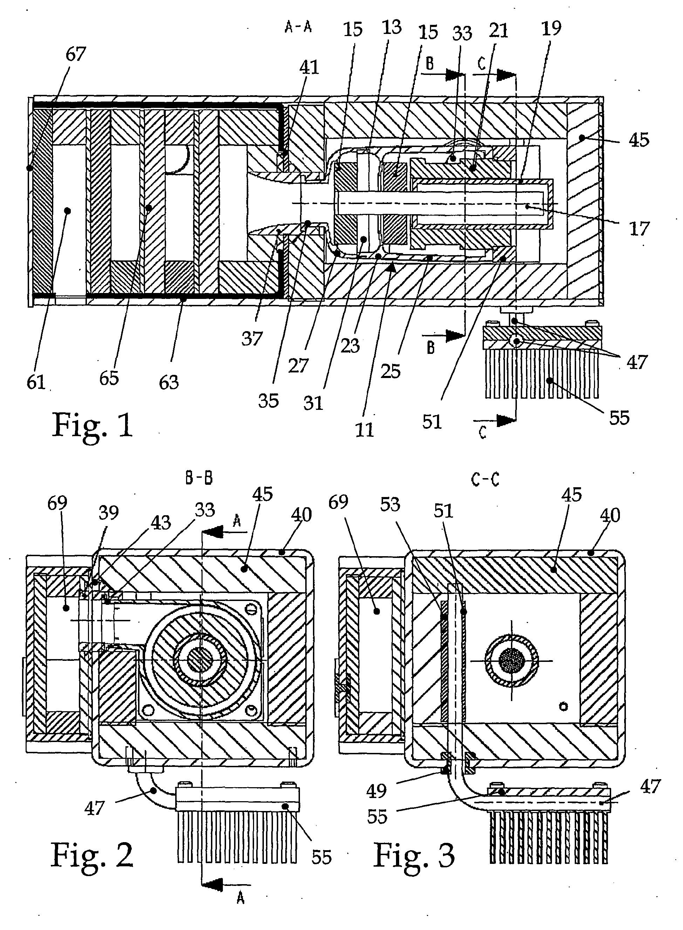

Das in den Figuren 1 bis 3 dargestellte Gebläse 11 ist folgendermassen aufgebaut. In einem Gebläsegehäuse 13 sind zwei Kompressions-Räder 15 auf einer gemeinsamen Welle 17 angeordnet. Die Welle 17 ist durch einen Elektromotor 19 angetrieben. Der Elektromotor 19 ist in einem Aluminiumprofil eingefasst, welche eine innere Gehäusewandung 21 des Gebläsegehäuses 13 bildet. Der Elektromotor 19 ist in thermischer Verbindung mit dieser inneren Gehäusewandung, da er mit dieser inneren Gehäusewandung 21 in grossflächiger Berührung steht. Diese innere Gehäusewandung 21 aus Aluminium und eine äussere Gehäusewandung 23 aus Kunststoff bilden zusammen das Gebläsegehäuse 13. Die äussere Gehäusewandung 23 ist aus einem Ausgangsteil 25 und einem Eingangsteil 27 zusammengesetzt. Der Ausgangsteil 25 umfasst mit den Kompressionsrädern 15 zusammenwirkende Statoren 31 und die Ausblasöffnung 33. Das Eingangsteil 27 umfasst die Ansaugöffnung 35 und einen Abschluss. An die Ansaugöffnung 35 ist ein Formteil 37 aus Gummi oder einem ähnlichen elastischen Material angeordnet, das den Kompressoreingang aerodynamisch ausformt und gleichzeitig das Gebläsegehäuse vibrationssdämpfend an einer Tragwand 41 abstützt. Ebenso ist an der Ausblasöffnung 33 des Gebläsegehäuses 13 ein zweites Formteil 39 angeordnet, welches Formteil 39 (Fig. 2) das Gebläsegehäuse 13 ebenfalls vibrationsdämpfend an einer Wand 43 abstützt.

Die Wand 43 ist Teil eines Aluminiumbehälters 40, in dessen Innerem die Tragwand 41 und das Gebläse 11 angeordnet ist. Zwischen dem Gebläsegehäuse 13 und dem Elektromotor 19 einerseits und diesem Aluminiumbehälter 40 andererseits ist eine Schall dämmende und absorbierende Schicht 45 angeordnet. Diese Schicht 45 umschliesst das gesamte Gebläse mit Gebläsegehäuse und Motor. Sie wird durch die Ansaugöffnung 35 und die Ausblasöffnung 33 durchdrungen. Sie wird zudem durch eine Heatpipe 47 durchdrungen (Fig. 3). Diese Heatpipe 47 ist beim Durchgang durch die Wand des Aluminiumbehälters 40 mit einer Gummimuffe 49 eingefasst.

Diese Heatpipe bildet ein für die Erfindung wesentliches Teil. Um ihre Aufgabe und Anordnung besser zu verstehen, muss nochmals zurück zur Figur 1 gegangen werden.

Das Aluminiumprofil, das eine innere Gehäusewandung 21 bildet, ist zwischen dem Motor und den diesen umgebenden Luftwegen angeordnet. Im Innern dieser inneren Gehäusewandung 21 entwickelt sich beim Betrieb des Gebläses Wärme als Abwärme des Elektromotors. Ausserhalb dieser inneren Gehäusewandung 21 fällt ebenfalls Wärme an. Diese Wärme entsteht durch die Kompression des Beatmungsgases. Diese komprimierte und dadurch erhitzte Luft streicht an dieser inneren Gehäusewandung 21 entlang.

Um diese Wärme abführen zu können, wird erfindungsgemäss die innere Gehäusewandung 21 gekühlt. Um dies zu erreichen ist ein Flansch 51 an der Gehäusewandung 21 angeordnet. Zwischen diesem Flansch 51 und einem einstückig mit diesem ausgebildeten Klemmteil 53 (Fig. 3) ist die Heatpipe 47 geklemmt. Bevorzugt wird ein Klemmsitz der Heatpipe in einer Bohrung im Flansch 51. Die Heatpipe führt daher Wärme von der inneren Gehäusewandung 21 ab. Sie führt durch die Schicht 45 und den Aluminiumbehälter 40 hindurch zu einem Kühlelement 55. Im Kühlelement 55 ist die Heatpipe 47 wieder geklemmt um einen möglichst guten Wärmeübergang zu gewährleisten. Das Kühlelement 55 ist mit Kühlstäbchen bestückt und aus Aluminium gefertigt.

Durch diese Anbindung einer Heatpipe an die innere Gehäusewandung 21 kann daher verhindert werden, dass
- 1. die Atemluft durch die Abwärme des Elektromotors erwärmt wird,
- 2. das Lagerfett des Elektromotors in Folge eines Hitzestaus überhitzt wird,

Es kann erreicht werden, dass
- 3. die Atemluft lediglich in angenehmem Mass erwärmt wird, und daher nicht zusätzlich gekühlt zu werden braucht,
- 4. die Lärmemissionen der Kompressionsräder und des Motors durch diese Teile umschliessende, Schall dämmende und absorbierende Schichten reduziert werden kann,
- 5. das Gebläse in einen festen Behälter eingepackt werden kann, an dem die vibrierenden Teile vibrationsdämpfend aufgehängt werden können,
- 6. die Lebensdauer des Geräts erhöht ist,
- 7. ein hochtouriger Motor eingesetzt werden kann,
- 9. das Gebläse in eine wärmedämmende Umgebung eingesetzt werden kann.

Der Behälter 40 wird in dieser Schrift auch als Aussenhülle bezeichnet. Diese Aussenhülle 40 enthält beim dargestellten Ausführungsbeispiel nicht lediglich das Gebläse und den Motor, sondern auch eine Schalldämmkammer 61. Die Schalldämmkammer kann in einem Einschubteil 63 ausgebildet oder fix integriert sein. In dieser Schalldämmkammer 61 sind Schickanen 65 eingebaut, um die die Luft oder das Beatmungsgas herumgeführt wird. Die Oberflächen und die Materialbeschaffenheit dieser Schickanen 65 gewährleisten, dass die über die einströmende Luft aus dem Gebläse hinausgetragene Lärmemission des Kompressors stark reduziert wird.

Die Aussenhülle oder der Behälter 40 ist mit einem Deckel 67 abgeschlossen, so dass das Einschubteil 63 an seinem bestimmungsgemässen Ort festgehalten ist.

Auch anschliessend an die Ausblasöffnung 33 ist ein Schallfalle angeordnet. Diese Schallfalle 69 ist ausserhalb der Aussenhülle 40 ausgebildet und ist wesentlich kleiner als die eingangsseitig ausgebildete Schalldämmkammer 61.

An dieser Schallfalle 69 ist eine Steckverbindung ausgebildet, mit welcher der Luftführungskanal des Gebläses 11 mit einem weiterführenden Luftkanal zusammengefügt werden kann.

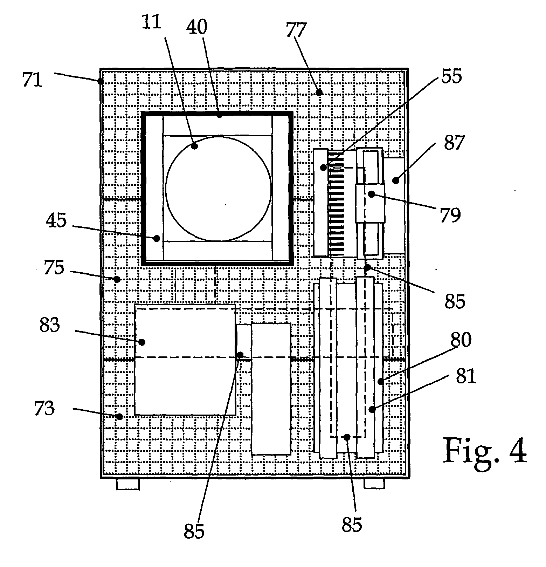

In Figur 4 ist das in der Aussenhülle 40 eingefasste Gebläse 11 in eingebauten Zustand dargestellt. Das Beatmungsgerät besitzt ein Gerätegehäuse 71. Dieses Gerätegehäuse 71 ist praktisch ausgefüllt mit drei Schaustoffteilen 73,75,77. In diesen Schaumstoffteilen sind Hohlräume 80 ausgespart, in denen die technisch notwendigen Bestandteile 11 (Gebläse), 40 (Aussenhülle des Gebläses), 55 (Kühlelement), 79 (Lüfter), 81 (Elektronische Steuerung), 83 (Ventile) etc. des Beatmungsgeräts wie zwischen Verpackungskörpern angeordnet sind. Diese Schaumstoffkörper 73, 75, 77 halten die technischen Bestandteile an der vorbestimmten Stelle und in einer konstanten Stellung zueinander fest. Zwischen den technischen Bestandteilen und den Schaumstoffkörpern sind Luftwege 85 ausgebildet. Diese besitzen einen Lufteinlass 87. Beim Lufteinlass ist ein Lüfter 79 angeordnet, der Luft in diese Luftwege 85 bläst. Diese Luft streicht z.B. zuerst am Kühlelement 55 vorbei und kühlt dieses und damit über die Heatpipe die innere Gehäusewandung des Gebläses.

Die Luftwege 85 führen danach durch die Hohlräume 80 mit den darin angeordneten elektronischen Schaltungen 81, den Ventilen 83 und so weiter bis zu einem (nicht dargestellten) Luftauslass.

## Patentansprüche

1. Gebläse (11) für ein Beatmungsgerät, das zur Kompression von Beatmungsgas mit einem Elektromotor (19) und mit wenigstens einem vom Elektromotor angetriebenen Kompressorrad (15) in einem Gebläsegehäuse (13) ausgerüstet ist, bei welchem Gebläse der Elektromotor (19) an einer ersten Gehäusewandung (21) des Gebläsegehäuses (13) anliegt, die aus einem gut wärmeleitenden Material, insbesondere Metall, vorzugsweise Aluminium, gefertigt ist, und bei welchem an die erste Gehäusewandung (21) unmittelbar angrenzend zwischen dieser ersten Gehäusewandung (21) und einer zweiten Gehäusewandung (25) wenigstens ein Luftführungskanal für das komprimierte Beatmungsgas vorhanden ist, **dadurch gekennzeichnet, dass** die erste Gehäusewandung (21) des Gebläsegehäuses (13) an wenigstens eine Heatpipe (47) angeschlossen ist, welche Heatpipe (47) mit einem Kühlelement (55) verbunden ist.

2. Gebläse nach Anspruch 1, **dadurch gekennzeichnet, dass** die Heatpipe (47) eine Schalldämmschicht (45) durchdringt, die das Gebläsegehäuse (13) umschliesst und das Kühlelement (55) ausserhalb dieser Schalldämmschicht (45) angeordnet ist.

3. Gebläse nach Anspruch 2, **dadurch gekennzeichnet, dass** die Schalldämmschicht (45) die Baueinheit aus Elektromotor (19) und Gebläsegehäuse (13) allseitig umschliesst.

4. Gebläse nach Anspruch 2 oder 3, **dadurch gekennzeichnet, dass** die Schalldämmschicht (45) im Innern einer Aussenhülle (40) angeordnet ist, in welcher Aussenhülle (40) die erste (21) und die zweite Gehäusewandung (25) und der Elektromotor (19) angeordnet sind, und ausserhalb welcher Aussenhülle (40) das Kühlelement (55) angeordnet ist.

5. Gebläse nach einem der Ansprüche 1 bis 4, **dadurch gekennzeichnet, dass** auf der Unterdruckseite des Kompressorrads (15) eine Schalldämpf-Kammer (61) vorhanden ist.

6. Gebläse nach Anspruch 4 und 5, **dadurch gekennzeichnet, dass** die Schalldämpf-Kammer (61) innerhalb der Aussenhülle (40) ausgebildet ist.

7. Beatmungsgerät oder Anästhesiegerät mit einem Gebläse (11), das zur Kompression von Beatmungsgas mit einem Elektromotor (19) und wenigstens einem vom Elektromotor angetriebenen Kompressorrad (15) in einem Gebläsegehäuse (13) ausgerüstet ist, bei welchem Gebläse der Elektromotor (19) an einer ersten Gehäusewandung (21) des Gebläsegehäuses (13) anliegt, und bei welchem Gebläse an die erste Gehäusewandung (21) unmittelbar angrenzend zwischen dieser ersten Gehäusewandung (21) und einer zweiten Gehäusewandung (25) wenigstens ein Luftführungskanal für das komprimierte Beatmungsgas vorhanden ist, **dadurch gekennzeichnet, dass** die erste Gehäusewandung (21) des Gebläsegehäuses aus einem gut wärmeleitenden Material, insbesondere Metall, vorzugsweise Aluminium, gefertigt ist und an wenigstens eine Heatpipe (47) angeschlossen ist, welche Heatpipe (47) mit einem Kühlelement (55) verbunden ist.

8. Gerät nach Anspruch 7, **gekennzeichnet durch** wenigstens einen Schaumstoffkörper (73,75,77), in welchem das Gebläse eingebettet ist.

9. Gerät nach Anspruch 8, **dadurch gekennzeichnet, dass** im Innern einer Aussenhülle (40) eine Schalldämmschicht (45) die erste (21) und die zweite Gehäusewandung (25) und der Elektromotor (19) angeordnet sind, und ausserhalb dieser Aussenhülle (40) das Kühlelement (55) angeordnet ist, und dass die Aussenhülle (40) im Schaumstoffkörper (75,77) eingebettet ist.

10. Gerät nach Anspruch 8 oder 9, **dadurch gekennzeichnet, dass** in diesem wenigstens einen Schaumstoffkörper (73,75,77) weitere technische Bestandteile (79, 81, 83) des Beatmungsgeräts oder Anästhesiegeräts angeordnet sind und vom Schaumstoffkörper (72,75,77) in der zu den anderen Bestandteilen (40,55,79,81,83) richtigen Position gehalten sind.

11. Gerät nach Anspruch 10, **dadurch gekennzeichnet, dass** ein Lüfter (79) vorhanden ist und zwischen den technischen Bestandteilen (40,55,79,81,83) des Beatmungs- oder Anästhesiegeräts und dem Schaumstoffkörper (73,75,77) ein Kühlluft-Kanalsystem (80) ausgebildet ist, das wenigstens einen Lufteinlass (79) und wenigstens einen Luftauslass besitzt.

12. Gerät nach Anspruch 11, **dadurch gekennzeichnet, dass** das Kühlelement (55) in diesem Kühlluft-Kanalsystem (80) angeordnet ist.

13. Gerät nach einem der Ansprüche 10 bis 12, **Dadurch gekennzeichnet, dass** mehrere Schaumstoffkörper (73,75,77) durch ein sie umschliessendes Gerätegehäuse (71) zusammengehalten sind und **dadurch** die zwischen den Schaumstoffkörpern angeordneten technischen Bestandteile des Beatmungsgeräts verschiebefest zwischen den Schaumstoffkörpern festgehalten sind.

## Claims

1. A blower (11) for a respiration apparatus, which is designed for the compression of respiration gas, with an electric motor (19) and with at least one compressor wheel (15) which is driven by the elector motor (1), in a blower housing (13), with which blower the electric motor (19) bears on a first housing wall (21) of the blower housing (13), said first housing wall being manufactured of a good heat-conducting material, in particular metal, preferably aluminium, and with which at least one air duct for the compressed respiration gas is present directly adjacent the first housing wall (21), between this first housing wall (21) and a second housing wall (25), **characterised in that** the first housing wall (21) of the blower housing (13) is connected to at least one heat pipe (47), said heat pipe (47) being connected to a cooling element (55).

2. A blower according to claim 1, **characterised in that** the heat pipe (47) penetrates a sound insulation layer (45), which encloses the blower housing (13), and the cooling element (55) is arranged outside this sound insulation layer (45).

3. A blower according to claim 2, **characterised in that** the sound insulation layer (45) encloses the construction unit of the electric motor (19) and the blower housing (13), on all sides.

4. A blower according to claim 2 or 3, **characterised in that** the sound insulation layer (45) is arranged in the inside of an outer casing (40), in which outer casing (40) the first (21) and the second housing wall (25) and the electric motor (19) are arranged, and the cooling element (55) is arranged outside said outer casing (40).

5. A blower according to one of the claims 1 to 4, **characterised in that** a sound damping chamber (61) is present on the vacuum side of the compressor wheel (15).

6. A blower according to claim 4 and 5, **characterised in that** the sound damping chamber (61) is formed within the outer casing (40).

7. A respiration apparatus or anesthesia apparatus with a blower (11), which is designed for the compression of respiration gas, with an electric motor (19) and with at least one compressor wheel (15) which is driven by the electric motor (1), in a blower housing (13), with which blower the electric motor (19) bears on a first housing wall (21) of the blower housing (13), and with which blower at least one air duct for the compressed respiration gas is present directly adjacent this first housing wall (21), between this first housing wall (21) and a second housing wall (25), **characterised in that** the first housing wall (21) of the blower housing is manufactured of a good heat-conducting material, in particular metal, preferably aluminium, and is connected to at least one heat pipe (47), said heat pipe (47) being connected to a cooling element (55).

8. An apparatus according to claim 7, **characterised by** at least one foam body (73, 75, 77), in which the blower is embedded.

9. An apparatus according to claim 8, **characterised in that** a sound insulation layer (45) , the first (21) and the second housing wall (25) and the electric motor (19) are arranged in the inside of an outer casing (40), and the cooling element (55) is arranged outside this outer casing (40) and that the outer casing (40) is embedded in the foam body (75, 77).

10. An apparatus according to claim 8 or 9, **characterised in that** further technical components (79, 81, 83) of the respiration apparatus or anesthesia apparatus are arranged in this at least one foam body (73, 75, 77), and are held by the foam body (72, 75, 77) in the correct position to the other components (40, 55, 79, 81, 83).

11. An apparatus according to claim 10, **characterised in that** a fan (79) is present and a cooling air channel system (80) is designed between the technical components (40, 55, 79, 81, 83) of the respiration apparatus or anesthesia apparatus and the foam body (73, 75, 79), said cooling air channel system having at least one air inlet (79) and at least one air outlet.

12. An apparatus according to claim 11, **characterised in that** the cooling element (55) is arranged in this cooling air channel system (80).

13. An apparatus according to one of the claims 10 to 12, **characterised in that** several foam bodies (73, 75, 77) are held together by way of an apparatus housing (71) enclosing it, and by way of this, the technical components of the respiration apparatus which are arranged between the foam bodies, are held in a fixed manner between the foam bodies in a dislocation-proof manner.

## Revendications

1. Soufflante (11) pour un respirateur qui est équipée, pour la compression du gaz respiratoire, d'un moteur électrique (19) et d'au moins une roue de compresseur (15) entraînée par un moteur électrique dans un bâti de soufflante (13), soufflante pour laquelle le moteur électrique (13) s'appuie sur une première paroi de bâti (21) du bâti de soufflante (13) qui est fabriquée en un matériau ayant une bonne conduction thermique, en particulier en métal, de préférence en aluminium, et pour laquelle il existe au moins un conduit de conduction d'air pour le gaz respiratoire comprimé directement adjacent à la première paroi du bâti (21) entre cette première paroi de bâti (21) et une seconde paroi du bâti (25), **caractérisée en ce que** la première paroi de bâti (21) du bâti de soufflante (13) est raccordée à au moins un tube de chauffe (47), lequel tube de chauffe (47) est relié à un élément de refroidissement (55).

2. Soufflante selon la revendication 1, **caractérisée en ce que** le tube de chauffe (47) traverse une couche d'isolation acoustique (45) qui entoure le bâti de soufflante (13) et l'élément de refroidissement (55) à l'extérieur de cette couche d'isolation acoustique (45).

3. Soufflante selon la revendication 2, **caractérisée en ce que** la couche d'isolation acoustique (45) entoure l'unité composée du moteur électrique (19) et du bâti de soufflante (13) de tous les côtés.

4. Soufflante selon la revendication 2 ou 3, **caractérisée en ce que** la couche d'isolation acoustique (45) est placée à l'intérieur d'une enveloppe extérieure (40), enveloppe extérieure (40) dans laquelle la première (21) et la seconde paroi de bâti (25) et le moteur électrique (19) sont placés et enveloppe extérieure (40) à l'extérieur de laquelle l'élément de refroidissement (55) est placé.

5. Soufflante selon l'une des revendications 1 à 4, **caractérisée en ce qu'**il existe une chambre d'isolation acoustique (61) sur le côté dépression de la roue du compresseur (15).

6. Soufflante selon la revendication 4 et 5, **caractérisée en ce que** la chambre d'isolation acoustique (61) est configurée à l'intérieur de l'enveloppe extérieure (40).

7. Respirateur ou appareil d'anesthésie avec une soufflante (11) qui est équipée, pour la compression du gaz respiratoire, d'un moteur électrique (19) et d'au moins une roue de compresseur (15) entraînée par un moteur électrique dans un bâti de soufflante (13), soufflante pour laquelle le moteur électrique (13) s'appuie sur une première paroi de bâti (21) du bâti de soufflante (13) et pour laquelle il existe au moins un conduit de conduction d'air pour le gaz respiratoire comprimé de ventilation directement adjacent à la première paroi du bâti (21) entre cette première paroi de bâti (21) et une seconde paroi du bâti (25), **caractérisé en ce que** la première paroi de bâti (21) du bâti de soufflante est fabriquée en un matériau ayant une bonne conduction thermique, en particulier en métal, de préférence en aluminium et est raccordée à au moins un tube de chauffe (47), lequel tube de chauffe (47) est relié à un élément de refroidissement (55).

8. Appareil selon la revendication 7, **caractérisé par** au moins un corps en mousse (73, 75, 77) dans lequel la soufflante est encastrée.

9. Appareil selon la revendication 8, **caractérisé en ce qu'**une couche d'isolation acoustique (45), la première et la seconde paroi de bâti (25) et le moteur électrique (19) sont placées dans l'intérieur d'une enveloppe extérieure (40) et que l'élément de refroidissement (55) est placé à l'extérieur de cette enveloppe extérieure (40) et que l'enveloppe extérieure (40) est encastrée dans le corps en mousse (75, 77).

10. Appareil selon la revendication 8 ou 9, **caractérisé en ce que** d'autres composants techniques (79, 81, 83) du respirateur ou de l'appareil d'anesthésie sont placés dans ce corps en mousse (73, 75, 77) qui existe au moins et qu'ils sont maintenus dans la position correcte par rapport aux autres composants (40, 55, 79, 81, 83) par le corps en mousse (72, 75, 77).

11. Appareil selon la revendication 10, **caractérisé en ce qu'**il est existe un aérateur (79) et qu'un système de conduit d'air de refroidissement (80), qui possède au moins une entrée d'air (79) et au moins une sortie d'air, est configuré entre les composants techniques (40, 55, 79, 81, 83) du respirateur ou de l'appareil d'anesthésie et le corps en mousse (73, 75, 77).

12. Appareil selon la revendication 11, **caractérisé en ce que** l'appareil de refroidissement (55) est placé dans ce système de conduit d'air de refroidissement (80).

13. Appareil selon l'une des revendications 10 et 12, **caractérisé en ce que** plusieurs corps en mousse (73, 75, 7) sont maintenus ensemble par un bâti d'appareil (71) qui les entoure et que les composants techniques du respirateur qui sont placés entre les corps en mousse sont maintenus résistants au déplacement entre les corps en mousse.
